Europäisches Patentamt

European Patent Office

Office européen des brevets

(19)

(11) Veröffentlichungsnummer : **0 384 198 B1**

(12) # EUROPÄISCHE PATENTSCHRIFT

(45) Veröffentlichungstag der Patentschrift :
**29.12.93 Patentblatt 93/52**

(51) Int. Cl.$^5$ : **C01C 1/24,** C07C 253/34,
C07C 255/08

(21) Anmeldenummer : **90102176.6**

(22) Anmeldetag : **03.02.90**

(54) **Verfahren zur Entfernung von Ammoniumsulfat aus hochteerhaltigen Abfallströmen der (Meth) Acrylnitrilherstellung.**

(30) Priorität : **18.02.89 DE 3905087**
**15.07.89 DE 3923423**

(43) Veröffentlichungstag der Anmeldung :
**29.08.90 Patentblatt 90/35**

(45) Bekanntmachung des Hinweises auf die
Patenterteilung :
**29.12.93 Patentblatt 93/52**

(84) Benannte Vertragsstaaten :
**DE ES FR GB IT**

(56) Entgegenhaltungen :
**EP-A- 0 126 058**
**DE-A- 1 952 480**
**US-A- 3 408 157**
**US-A- 3 902 859**
**CHEMICAL ABSTRACTS Band 88, 1978, Zu-**
**sammenfassung Nr. 123360s, Columbus, Ohio,**
**US; & JP-A-52153899 (UBE Industries)**
**21.12.1977**

(73) Patentinhaber : **EC ERDÖLCHEMIE GMBH**
**Alte Strasse 201**
**D-50769 Köln (DE)**

(72) Erfinder : **Haardt, Hans-Jürgen, Dr.**
**Rur-Strasse 3**
**D-5024 Pulheim (DE)**
Erfinder : **Herwig, Jens, Dr.**
**Nerzweg 2**
**D-5000 Köln 40 (DE)**
Erfinder : **Neeb, Ernst-Friedrich, Dr.**
**Kronenpützchen 28**
**D-4047 Dormagen 11 (DE)**

(74) Vertreter : **Müller, Gerhard, Dr. et al**
**BAYER AG Konzernverwaltung RP Patente**
**Konzern**
**D-51368 Leverkusen (DE)**

Jouve, 18, rue Saint-Denis, 75001 PARIS

EP 0 384 198 B1

**Beschreibung**

Das erfindungsgemäße Verfahren betrifft die Entfernung von Ammoniumsulfat aus hochteerhaltigen Abfallströmen der (Meth)Acrylnitrilherstellung durch Zusatz von Methanol und Ammoniak und Abtrennung des dabei ausfallenden Ammoniumsulfats. Der zurückbleibende Abfallstrom ist dadurch von seinem Schwefelgehalt weitgehend befreit.

Im Bestreben, die Emission von umweltbelastenden Gasen, zu denen auch $SO_2$ gehört, zu minimieren, sind Gesetze und Verordnungen verabschiedet worden, die Emissionsgrenzwerte festlegen. So ist beispielsweise für die Bundesrepublik Deutschland in der "Technischen Anleitung Luft" (TA Luft) bestimmt, daß der $SO_2$-Gehalt im Rauchgas von Verbrennungsanlagen den Wert von 100 mg/Nm³ nicht überschreiten darf. Der entsprechende Grenzwert für Stickoxide ($NO_x$) liegt bei 500 mg/Nm³.

Durch den Einsatz schwefelhaltiger (Abfall-)Brennstoffe werden vielfach $SO_2$-Konzentrationen von mehr als 1000 mg/Nm³ im Rauchgas erhalten. Zur Entfernung des $SO_2$ ist deshalb beispielsweise in Kohlekraftwerken als zusätzliche Verfahrensstufe eine Rauchgasentschwefelung installiert, in der nach einer aufwendigen Gaswäsche das $SO_2$ mit Kalk zu Gips ($CaSO_4$) umgesetzt wird. Durch den steigenden Anfall von Gips wird dessen sinnvolle Entsorgung, beispielsweise als Baustoffkomponente, zunehmend schwieriger. Sowohl Investitionen als auch Betriebskosten (Einsatz von Kalk u.a.) und Gipsentsorgung stellen eine hohe Belastung für die Energiekosten (öffentliche Energieversorgung) bzw. Produktionskosten (Industriekraftwerke) dar.

Um diesen Aufwand einer umweltgerechten Entsorgung zu minimieren oder gar entbehrlich zu machen, sollte das Ziel verfolgt werden, entweder den Anfall der Schadstoffe zu verhindern oder, wenn ihre Entstehung nicht zu vermeiden ist, ihre umweltgerechte Entfernung möglichst frühzeitig, d.h. direkt nach ihrem Anfall, vorzunehmen. Eine solche Lösung des Entsorgungsproblems von ammoniumsulfathaltigen Abfallströmen der (Meth)Acrylnitrilproduktion wird durch das erfindungsgemäße Verfahren erreicht.

Bei der Herstellung von Acrylnitril oder Methacrylnitril durch Ammonoxidation von Propylen bzw. i-Buten muß überschüssiges Ammoniak aus dem Reaktionsgas entfernt werden. Durch Zugabe von Schwefelsäure wird das Ammoniak zu Ammoniumsulfat umgesetzt; dies verbleibt entweder in der Sulfatlauge oder fällt als abzutrennender Kristallbrei aus.

In dieser Sulfatlauge sind auch die bei der (Meth)Acrylnitrilherstellung unvermeidlichen Oligomeren und Polymeren enthalten.

Es ist bereits versucht worden (EP 126 058), die Mutterlauge der Ammoniumsulfat-Kristallisation mit Methanol zu behandeln; als Ziel wurde hierbei die Gewinnung von weiterem qualitativ befriedigenden Ammoniumsulfat angesehen. Hierzu soll der Teergehalt (Oligomere und Polymere) höchstens 25 %, bevorzugt höchstens 20 %, betragen. Dadurch liegt eine absolut größere Menge an Wasser und Ammoniumsulfat vor. Die zur grundsätzlich bekannten Salzausfällung benötigte Methanolmenge steigt dadurch ebenfalls stark an. Dennoch gelingt es nicht, den restlichen Salzgehalt und damit den restlichen Schwefelgehalt soweit zu drücken, daß bei einer Verbrennung der hierbei verbleibenden Restlauge der $SO_2$-Gehalt der Verbrennungsgase den Bestimmungen der TA Luft genügt. Im Verfahren dieser EP 126 058 wird das so gewonnene Ammoniumsulfat mit roher Sulfatlauge aufgelöst und wieder Hauptkristallisation zugeführt, während die gesamte aus der Methanolbehandlung stammende Restlauge (nach Methanol-Rückgewinnung) einer Verbrennung zugeführt wird und diese belastet.

Es wurde nun gefunden, daß es vorteilhafter ist, die Mutterlauge aus der Hauptkristallisation, wie sie nach der Ammoniumsulfat-Abtrennung beispielsweise als Zentrifugat vorliegt, durch Absitzenlassen in einen hochteerhaltige Abfallstrom und eine im wesentlichen teerfreie Mutterlauge zu trennen.

Dieser hochteerhaltige Abfallstrom kann von der Oberfläche der Sulfatlauge beispielsweise durch Abskimmen abgetrennt werden und im erfindungsgemäßen Verfahren behandelt werden. Solche Abfallströme enthalten zwischen 25 und 80 Gew.-% Oligomere und/oder Polymere aus der (Meth)Acrylnitrilherstellung, typischerweise 30-60 Gew.-%. Da diese teerhaltigen Abfallströme mit der Ammoniumsulfatlauge in Kontakt gestanden haben, enthalten sie noch Ammoniumsulfat in einer Menge von 10-30 Gew.-%, typischerweise 15-25 Gew.-%; bei einer Entsorgung solcher Abfallströme in einer Verbrennungsanlage ohne Vorbehandlung tragen diese Abfallströme deshalb zur Erhöhung der $SO_2$- und $NO_x$-Emission bei. Der Rest zu 100 % in diesen Abfallströmen ist im wesentlichen Wasser. Abfallströme der genannten Art fallen in einer Menge von 20-80 kg/t (Meth)Acrylnitril, typischerweise 25-40 kg/t, als Abfallprodukt an.

Die beispielsweise durch Abskimmen befreite Mutterlauge wird in die Hauptkristallisation zurückgeführt.

Das erfindungsgemäße Verfahren erlaubt nun, nahezu den gesamten Schwefelgehalt und weiterhin den überwiegenden Stickstoffgehalt solcher Abfallströme zu entfernen und damit diese Abfallströme bei ihrer Entsorgung leichter handhabbar zu machen.

Es wurde ein Verfahren zur Entfernung von Ammoniumsulfat aus hochteerhaltigen Abfallströmen der (Meth)Acrylnitrilherstellung, die durch Abtrennung von der Mutterlauge der $(NH_4)_2SO_4$-Hauptkristallisation er-

2

EP 0 384 198 B1

halten werden und die einen Teergehalt von 25-80 Gew.-% haben, gefunden, das dadurch gekennzeichnet ist, daß man solche Abfallströme bei 10-60° C mit der 1- bis 30-fachen Gewichtsmenge Methanol, bezogen auf den Wassergehalt des Abfallstroms, und mit 0,05 - 10 Gew.-% Ammoniak, bezogen auf die Methanolmenge, versetzt und das ausfallende Ammoniumsulfat abtrennt.

Methanol wird in der 1- bis 30-fachen Gewichtsmenge, bevorzugt der 3- bis 20-fachen Gewichtsmenge, bezogen auf das im Abfallstrom enthaltene Wasser, eingesetzt.

Ammoniak wird dem Abfallstrom in einer Menge van 0,05-10 Gew.-%, bezogen auf die Methanolmenge zugesetzt werden. Bevorzugt wird Ammoniak in der 0,1 - 8 %igen Gewichtsmenge, besonders bevorzugt der 0,2 - 6 %igen Gewichtsmenge, bezogen auf Methanol, zugesetzt. Dieser Zusatz kann durch Einführen von gasförmigem oder flüssigem Ammoniak oder durch Zusatz von ammoniakhaltigem Wasser erfolgen. Das gasförmige Ammoniak oder ein ammoniakhaltiges Wasser können auch Abfallströme aus der (Meth)-Acrylnitrilherstellung oder geeignete Abfallströme aus einem anderen Produktionsverfahren sein. Die Rolle des Ammoniaks entspricht einem Vorstellungsmodell, nach dem die Oligomeren und Polymeren, möglicherweise durch Solvatation, "aufgeschlossen" werden, wodurch der Einschluß und das übermäßige Verbleiben von Ammoniumsulfat verhindert werden Außerdem kann durch den Ammoniakzusatz der Anteil von Polymeren im gefällten Ammoniumsulfat minimiert werden.

Das erfindungsgemäße Verfahren wird bei 10 bis 60°C, bevorzugt bei 25 bis 45°C durchgeführt. Das Verfahren kann diskontinuierlich oder kontinuierlich, bevorzugt kontinuierlich, in einem Verweilzeitkessel, einem Strömungsrohr oder anderen, dem Fachmann geläufigen Reaktionsapparaten durchgeführt werden. Die Verweilzeiten für die Kristallisation und das Absetzen des abzutrennenden Ammoniumsulfats sollen mindestens 0,1 min. betragen und brauchen 50 min nicht zu überschreiten. Für die kontinuierliche Verfahrensdurchführung sind Verweilzeiten von 5 bis 50 min. geeignet. Die Durchführung des erfindungsgemäßen Verfahrens ist vom äußeren Druck unabhängig. Es kann daher bei Normaldruck, erhöhtem oder vermindertem Druck gearbeitet werden. Zur Vereinfachung des Verfahrens und der Reaktionsapparate wird im allgemeinen bei Normaldruck gearbeitet.

Nachdem der hochteerhaltige Abfallstrom mit dem Methanol sowie dem Ammoniak in Kontakt gebracht worden ist, wird das ausgefallene Ammoniumsulfat durch geeignete Weise, wie Filtrieren, Abzentrifugieren, Abdekantieren oder ähnliche geeignete Operationen abgetrennt und in einem Trockner von anhaftendem Wasser, Methanol sowie Ammoniak befreit. Danach kann dieses Ammoniumsulfat als Feststoff oder in Wasser aufgeschlämmt dem Ammoniumsulfatteil der (Meth)Acrylnitril-Produktionsanlage zugeführt werden. Das erfindungsgemäß gewonnene Ammoniumsulfat ist von einem hohen Reinheitsgrad, der eine Vielzahl von Verwendungen, beispielsweise als Düngemittel oder als Düngemittelkomponente, ermöglicht.

Der nach der Abtrennung von Ammoniumsulfat verbleibende Abfallstrom ist eine homogene flüssige Phase. Er kann in geeigneter Weise entsorgt werden, beispielsweise durch weitgehendes Eindampfen und Verbringen des Rückstandes auf eine Deponie oder, in bevorzugter Weise, durch Verbrennen in einer geeigneten Verbrennungskammer als schwefelarmer Abfallbrennstoff. Bei ausreichend hohem Brennwert eines solchen Abfallstromes kann dieser sogar noch gewinnbringend in einem Kraftwerk verwendet werden. Vor einer der genannten Entsorgungsarten können dem erfindungsgemäß anfallenden, von Ammoniumsulfat freien Abfallstrom auch weitere Abfallströme, beispielsweise aus der (Meth)Acrylnitrilherstellung, zugesetzt werden.

In einer weiteren Ausgestaltung des erfindungsgemäßen Verfahrens wird der nach der Abtrennung des ausgefallenen Ammoniumsulfats verbleibende Abfallstrom einer Destillationskolonne zugeführt, wo er vom Methanol, welches als Kopfprodukt aufgefangen wird, befreit wird. Gleichzeitig entweicht vorhandenes Ammoniak über Kopf und findet sich im kondensierten Methanol. Dieses kondensierte, Ammoniak enthaltende Methanol wird bevorzugt recyclisiert, d.h. erneut zur Ausfällung von Ammoniumsulfat erfindungsgemäß eingesetzt. Gegebenenfalls brauchen Methanol sowie Ammoniak nur im Maße ihrer unvermeidlichen Verluste ersetzt zu werden. Um den Sumpfumlauf einer solchen Destillationskolonne homogen, fließfähig und damit pumpfähig zu halten, wird vorteilhafterweise Wasser zum Sumpfumlauf in einer solchen Menge gegeben, daß der Wassergehalt des Sumpfumlaufes 10 - 50 Gew.-% des gesamten Sumpfumlaufs, beträgt. Es hat sich als besonders vorteilhaft erwiesen, wenn dieses zuzusetzende Wasser Ammoniak enthält. Dieser Ammoniakgehalt im zuzusetzenden Wasser beträgt 0,1 - 2 Gew.-% des zuzusetzenden Wassers und wird zum größten Teil ebenfalls über Kopf entfernt, bevor der im Sumpf umlaufende restliche Abfallstrom letztendlich zur Entsorgung abgezogen wird. Das dem Sumpfumlauf zuzusetzende, bevorzugt Ammoniak enthaltende Wasser kann ein geeigneter wäßriger Stoffstrom oder Abfallstrom der (Meth)Acrylnitril-Produktionsanlage sein. Solche Stoffströme und Abfallströme sind dem mit der (Meth)Acrylnitril-Herstellung vertrauten Fachmann bekannt. Dieser Zusatz zum Sumpfumlauf der genannten Destillationskolonne bietet somit eine weitere Möglichkeit der Vereinigung von Abfallströmen und damit ihrer vereinfachten Entsorgung.

Dem Sumpfumlauf wird bei kontinuierlicher Verfahrensführung kontinuierlich ein Anteil entnommen, der weitgehend vom Methanol und weitgehend vom Ammoniak befreit ist. Er wird in der oben beschriebenen Weise entsorgt. Da dieser dem Sumpfumlauf entnommene Abfallstrom nicht nur zuvor vom Ammoniumsulfat und damit von der $SO_2$-Quelle befreit worden war, sondern während der Destillation auch weitgehend vom Ammoniak befreit wurde, stellt er einen Abfallbrennstoff dar, der nicht nur schwefelarm, sondern auch stickstoffarm ist. Seine bevorzugte Entsorgung ist daher die Verbrennung in einer geeigneten Verbrennungskammer, beispielsweise durch Zuführung zum Kraftwerk. Falls erforderlich, kann ein solcher Abfallstrom vor der Verbrennung eingeengt und damit von einem Teil des Wassers befreit werden.

Die Entfernung des Ammoniumsulfats im erfindungsgemäß zu behandelnden Abfallstrom gelingt mindestens zu 95 % des ursprünglichen Wertes, in vielen Fällen zu mehr als 98 % bis hin zu 99 %. Die $SO_2$-Konzentration im Rauchgas bei der Verbrennung des erfindungsgemäß behandelten Abfallstroms liegt unter 100 mg/Nm³.

### Beispiele 1 bis 28

In einem offenen 250 ml-Becherglas wurden ammonsulfathaltiger Teer aus der Acrylnitrilproduktion, Methanol und Ammoniak in den Mengen und Zusammensetzungen, die in der Tabelle aufgeführt sind, unter Rühren zusammengegeben.

Der bei den jeweiligen Temperaturen ausgefällte Feststoff wurde abfiltriert, getrocknet und ebenso wie das Filtrat analysiert. Aus den Stoffbilanzdaten wurde für die Ammonsulfatausfällung der Entschwefelungsgrad bestimmt.

EP 0 384 198 B1

Tabelle: $(NH_4)_2SO_4$-Entfernung   (Beispiele 1 bis 28)

| Beisp.-Nr. | | 1 | 2 | 3 | 4 | 5 | 6 |
|---|---|---|---|---|---|---|---|
| Teer-Einsatz | g | 50 | 50,1 | 50,98 | 52,21 | 50,22 | 50,64 |
| S-Gehalt im Teer | % | 6,055 | 6,055 | 6,055 | 3,032 | 3,032 | 3,032 |
| Ammonsulfat d.Th. | g | 12,48 | 12,50 | 12,72 | 6,52 | 6,28 | 6,33 |
| Wasser i. Orig.Teer | % | 34,88 | 34,88 | 34,88 | 20,46 | 20,46 | 20,46 |
| Wasser i. Einsatzteer | % | 34,88 | 34,88 | 34,88 | 20,46 | 20,46 | 20,46 |
| Polymer i. Orig.Teer | % | 40,17 | 40,17 | 40,17 | 67,05 | 67,05 | 67,05 |
| Polymer i. Einsatzteer | % | 40,17 | 40,17 | 40,17 | 67,05 | 67,05 | 67,05 |
| Methanol/Teer | | 1 | 2 | 3 | 2 | 3 | 4 |
| Methanol/Polymer | | 2,5 | 5,0 | 7,5 | 3,0 | 4,5 | 6,0 |
| Methanol/Wasser | | 3 | 6 | 9 | 10 | 15 | 20 |
| $NH_3$-Anteil i.Methanol | % | 0,5 | 0,5 | 0,5 | 0,1 | 0,1 | 0,1 |
| Methanol Temp. | °C | 20 | 20 | 20 | 20 | 20 | 20 |
| Mischungstemp. | °C | 30 | 30 | 30 | 30 | 30 | 30 |
| S-Menge i. Filtrat | g | 0,091 | 0,037 | 0,031 | 0,084 | 0,063 | 0,069 |
| Ammonsulf. + Polymer getr. | g | 13,09 | 13,95 | 15,69 | 21,72 | 22,04 | 22,42 |
| S-Entf. bez.auf Orig.Teer | % | 96,98 | 98,77 | 99,01 | 94,72 | 95,88 | 95,49 |

Tabelle: $(NH_4)_2SO_4$-Entfernung (Fortsetzung)

| Beisp.-Nr. | | 7 | 8 | 9 | 10 | 11 | 12 |
|---|---|---|---|---|---|---|---|
| Teer-Einsatz | g | 50,22 | 50,54 | 25,43 | 23 | 10,38 | 5,03 |
| S-Gehalt im Teer | % | 3,032 | 3,032 | 3,032 | 3,032 | 3,032 | 3,032 |
| Ammonsulfat d.Th. | g | 6,28 | 6,32 | 3,18 | 2,87 | 1,30 | 0,63 |
| Wasser i. Orig.Teer | % | 20,46 | 20,46 | 20,46 | 20,46 | 20,46 | 20,46 |
| Wasser i. Einsatzteer | % | 20,46 | 39,21 | 51,37 | 63,53 | 75,67 | 87,84 |
| Polymer i. Orig.Teer | % | 67,05 | 67,05 | 67,05 | 67,05 | 67,05 | 67,05 |
| Polymer i. Einsatzteer | % | 67,05 | 51,24 | 41,00 | 30,74 | 20,51 | 10,25 |
| Methanol/Teer | | 3 | 3 | 3 | 3 | 3 | 3 |
| Methanol/Polymer | | 4,5 | 5,9 | 7,3 | 9,8 | 14,6 | 29,3 |
| Methanol/Wasser | | 15 | 8 | 6 | 5 | 4 | 3 |
| $NH_3$-Anteil i.Methanol | % | 0,1 | 0,1 | 0,1 | 0,1 | 0,1 | 0,1 |
| Methanol Temp. | °C | 20 | 20 | 20 | 20 | 20 | 20 |
| Mischungstemp. | °C | 30 | 30 | 30 | 30 | 30 | 30 |
| S-Menge i. Filtrat | g | 0,063 | 0,124 | 0,074 | 0,115 | 0,098 | 0,110 |
| Ammonsulf. + Polymer getr. | g | 22,04 | 17,85 | 7,82 | 6,96 | 2,47 | 0,93 |
| S-Entf. bez.auf Orig.Teer | % | 95,88 | 91,92 | 90,40 | 83,48 | 68,96 | 27,77 |

EP 0 384 198 B1

EP 0 384 198 B1

Tabelle: $(NH_4)_2SO_4$-Entfernung (Fortsetzung)

| Beisp.-Nr. | | 13 | 14 | 15 | 16 | 17 | 18 |
|---|---|---|---|---|---|---|---|
| Teer-Einsatz | g | 50 | 50 | 50,98 | 50,5 | 50,66 | 50 |
| S-Gehalt im Teer | % | 6,055 | 6,055 | 6,055 | 6,055 | 5,443 | 5,443 |
| Ammonsulfat d.Th. | g | 12,48 | 12,48 | 12,72 | 12,60 | 11,36 | 11,22 |
| Wasser i. Orig.Teer | % | 34,88 | 34,88 | 34,88 | 34,88 | 39,44 | 39,44 |
| Wasser i. Einsatzteer | % | 34,88 | 34,88 | 34,88 | 34,88 | 39,44 | 39,44 |
| Polymer i. Orig.Teer | % | 40,17 | 40,17 | 40,17 | 40,17 | 38,13 | 38,13 |
| Polymer i. Einsatzteer | % | 40,17 | 40,17 | 40,17 | 40,17 | 38,13 | 38,13 |
| Methanol/Teer | | 3 | 3 | 3 | 3 | 3 | 3 |
| Methanol/Polymer | | 7,5 | 7,5 | 7,5 | 7,5 | 7,9 | 7,9 |
| Methanol/Wasser | | 9 | 9 | 9 | 9 | 8 | 8 |
| $NH_3$-Anteil i.Methanol | % | 0,5 | 0,5 | 0,5 | 0,5 | 0,5 | 0,5 |
| Methanol Temp. | °C | 2 | 10 | 20 | 30 | 2 | 10 |
| Mischungstemp. | °C | 22 | 27 | 30 | 40 | 24 | 28 |
| S-Menge i. Filtrat | g | 0,037 | 0,043 | 0,031 | 0,036 | 0,038 | 0,036 |
| Ammonsulf. + Polymer getr. | g | 23,2 | 15,29 | 15,69 | 14,73 | 16,27 | 15,83 |
| Polymer i. getr. Salz | % | 46,88 | 19,55 | 19,72 | 15,46 | 31,11 | 30,08 |
| S-Entf. bez.auf Orig.Teer | % | 98,78 | 98,59 | 99,01 | 98,82 | 98,63 | 98,69 |

Tabelle: $(NH_4)_2SO_4$-Entfernung (Fortsetzung)

| Beisp.-Nr. | | 19 | 20 | 21 | 22 | 23 | 24 |
|---|---|---|---|---|---|---|---|
| Teer-Einsatz | g | 50,35 | 50,22 | 55,3 | 50 | 50,98 | 50,15 |
| S-Gehalt im Teer | % | 5,443 | 5,443 | 5,443 | 6,055 | 6,055 | 6,055 |
| Ammonsulfat d.Th. | g | 11,29 | 11,27 | 12,40 | 12,48 | 12,72 | 12,51 |
| Wasser i. Orig.Teer | % | 39,44 | 39,44 | 39,44 | 34,88 | 34,88 | 34,88 |
| Wasser i. Einsatzteer | % | 39,44 | 39,44 | 39,44 | 34,88 | 34,88 | 34,88 |
| Polymer i. Orig.Teer | % | 38,13 | 38,13 | 38,13 | 40,17 | 40,17 | 40,17 |
| Polymer i. Einsatzteer | % | 38,13 | 38,13 | 38,13 | 40,17 | 40,17 | 40,17 |
| Methanol/Teer | | 3 | 3 | 3 | 3 | 3 | 3 |
| Methanol/Polymer | | 7,9 | 7,9 | 7,9 | 7,5 | 7,5 | 7,5 |
| Methanol/Wasser | | 8 | 8 | 8 | 9 | 9 | 9 |
| $NH_3$-Anteil i.Methanol | % | 0,5 | 0,5 | 0,5 | 1 | 0,5 | 0,25 |
| Methanol Temp. | °C | 21 | 30 | 40 | 20 | 20 | 20 |
| Mischungstemp. | °C | 35 | 42 | 50 | 30 | 34 | 30 |
| S-Menge i. Filtrat | g | 0,039 | 0,032 | 0,056 | 0,034 | 0,031 | 0,030 |
| Ammonsulf. + Polymer getr. | g | 14,59 | 15,08 | 19,11 | 14,65 | 15,69 | 15,69 |
| Polymer i. getr. Salz | % | 23,70 | 26,16 | 36,29 | 15,78 | 19,72 | 21,02 |
| S-Entf. bez.auf Orig.Teer | % | 98,56 | 98,84 | 98,15 | 98,88 | 99,01 | 99,03 |

EP 0 384 198 B1

**Tabelle: $(NH_4)_2SO_4$-Entfernung (Fortsetzung)**

| Beisp.-Nr. | | 25 | 26 | 27 | 28 |
|---|---|---|---|---|---|
| Teer-Einsatz | g | 50 | 50 | 50 | 50 |
| S-Gehalt im Teer | % | 6,055 | 5,071 | 3,071 | 5,071 |
| Ammonsulfat d.Th. | g | 12,48 | 10,45 | 10,45 | 10,45 |
| Wasser i. Orig.Teer | % | 34,88 | 49,64 | 49,64 | 49,46 |
| Wasser i. Einsatzteer | % | 34,88 | 53,8 | 60,35 | 65,27 |
| Polymer i. Orig.Teer | % | 40,17 | 28,46 | 28,46 | 28,46 |
| Polymer i. Einsatzteer | % | 40,17 | 26,11 | 22,41 | 19,63 |
| Methanol/Teer | | 3 | 2,8 | 2,4 | 2,2 |
| Methanol/Polymer | | 7,5 | 11,6 | 13,8 | 16,0 |
| Methanol/Wasser | | 9 | 6 | 5 | 5 |
| $NH_3$-Anteil i.Methanol | % | 0,1 | 1 | 3 | 5 |
| Methanol Temp. | °C | 20 | 20 | 20 | 20 |
| Mischungstemp. | °C | 30 | 30 | 30 | 30 |
| S-Menge i. Filtrat | g | 0,032 | 0,033 | 0,057 | 0,072 |
| Ammonsulf. + Polymer getr. | g | 17,13 | 13,22 | 13,15 | 13,16 |
| Polymer i. getr. Salz | % | 27,92 | 21,96 | 22,31 | 22,85 |
| S-Entf. bez.auf Orig.Teer | % | 98,96 | 98,71 | 97,76 | 97,17 |

### Beispiel 29

In einem 5m³-Rührkessel wurden 700 kg 7,4 % Schwefel enthaltender Teer mit 70°C aus der Acrylnitrilproduktion, 2,480 kg Methanol und 40 kg Ammoniakwasser (25 %ig) unter Rühren zusammengegeben. Das ausgefällte Ammonsulfat wurde abzentrifugiert und getrocknet. Der Zentrifugenablauf enthielt 0,99 % der ursprünglichen Schwefelmenge.

Das feuchte Salz mit einer Gesamtfeuchte von 43,25 % (Methanol : $H_2O$-Verhältnis ca. 3:1) konnte im Trockner bis zu einer Gesamtfeuchte von 2 - 4 % getrocknet werden. In dem getrockneten Ammonsulfat konnte kein Methanol mehr nachgewiesen werden.

Der Grad der Teerentschwefelung betrug ca. 99 %.

### Beispiel 30

2000 g heißer ammonsulfathaltiger Teer (70°C) aus der Acrylnitrilproduktion mit einem Schwefelgehalt von 4,7 Gew.-% wurden in einem 10 l Glasgefäß in 6000 g Methanol und 24 g $NH_3$-Wasser (25 %ig) eingerührt. Der dabei ausgefällte Feststoff wurde filtriert, mit wenig Methanol gewaschen und getrocknet. Es wurden 450 g Ammonsulfat erhalten.

Das Filtrat (6902 g) enthielt noch 1,712 g Schwefel, so daß der Entschwefelungsgrad 98,18 % betrug.

6700 g des Filtrats wurden mit 2523 g eines polymerhaltigen (30 Gew.-%) Wasserquenchstroms aus der Acrylnitrilproduktion gemischt und in einer Füllkörperkolonne von 1500 mm Länge und 30 mm Durchmesser mit Umlaufverdampfer und Kopfkondensation (Kühlmittel bei 4 - 5°C) kontinuierlich aufdestilliert.

Das 61 Gew.-% Methanol enthaltende Einsatzprodukt wurde in einer Menge von 547 g/h auf einer Höhe von 50 mm in die Kolonne gepumpt. Bei einem Rücklaufverhältnis von 3 : 1 wurden 311 g/h Kopfprodukt bei 66°C und 220 g/h Sumpfprodukt bei 102°C abgenommen. Das Sumpfprodukt war bei einer Viskosität von 0,93 mPas (90°C) gut pumpbar und enthielt noch 0,25 Gew.-% Restmethanol.

## Patentansprüche

1. Verfahren zur Entfernung von Ammoniumsulfat aus hochteerhaltigen Abfallströmen der (Meth)Acrylnitrilherstellung, die durch Abtrennung von der Mutterlauge der $(NH_4)_2SO_4$-Hauptkristallisation erhalten werden und die einen Teergehalt von 25-80 Gew.-% haben, dadurch gekennzeichnet, daß man solche Abfallströme bei 10 bis 60°C mit der 1 bis 30-fachen Gewichtsmenge Methanol, bezogen auf den Wassergehalt des Abfallstroms, und mit 0,05 -10 Gew.-% Ammoniak, bezogen auf die Methanolmenge, versetzt und das ausfallende Ammoniumsulfat abtrennt.

2. Verfahren nach Anspruch 1, dadurch gekennzeichnet, daß bei 25 bis 45° C gearbeitet wird.

3. Verfahren nach Anspruch 1, dadurch gekennzeichnet, daß die 3 bis 20-fache Gewichtsmenge an Methanol eingesetzt wird.

4. Verfahren nach Anspruch 1, dadurch gekennzeichnet, daß Ammoniak in einer Menge von 0,1 - 8 Gew.-%, bevorzugt von 0,2 - 6 Gew.-%, bezogen auf die Methanolmenge, zugesetzt wird.

5. Verfahren nach Anspruch 1, dadurch gekennzeichnet, daß der nach der Abtrennung des Ammoniumsulfats verbleibende Abfallstrom als schwefelarmer Abfallbrennstoff einer Verbrennungskammer zugeführt wird.

6. Verfahren nach Anspruch 1, dadurch gekennzeichnet, daß man das Methanol aus dem nach der Abtrennung des Ammoniumsulfats verbleibenden Abfallstrom gemeinsam mit dem Ammoniak abdestilliert.

7. Verfahren nach Anspruch 6, dadurch gekennzeichnet, daß das abdestillierte Methanol mit seinem Gehalt an Ammoniak erneut zur Entfernung von Ammoniumsulfat aus Abfallströmen eingesetzt wird.

8. Verfahren nach Anspruch 6, dadurch gekennzeichnet, daß der Sumpfstrom der Methanol-Destillation durch Zusatz von Wasser, bevorzugt durch Zusatz von Ammoniak enthaltendem Wasser, fließfähig gehalten wird.

9. Verfahren nach Anspruch 6, dadurch gekennzeichnet, daß der nach der Methanol-Destillation verbleibende Abfallstrom als schwefelarmer und stickstoffarmer Abfallbrennstoff einer Verbrennungskammer zugeführt wird.

10. Verfahren nach Anspruch 1, dadurch gekennzeichnet, daß es kontinuierlich durchgeführt wird.

## Claims

1. Process for the removal of ammonium sulphate from tar-rich waste streams from the production of (meth)acrylonitrile, which are obtained by separating off the mother liquor from the $(NH_4)_2SO_4$ main crystallization and have a tar content of 25 - 80 % by weight, characterized in that 1 to 30 times the amount by weight of methanol, relative to the water content of the waste stream, and 0.05 - 10% by weight of ammonia, relative to the amount of methanol, are added to such waste streams at 10 to 60°C, and the ammonium sulphate which precipitates out is separated off.

2. Process according to Claim 1, characterized in that the process is carried out at 25 to 45°C.

3. Process according to Claim 1, characterized in that 3 to 20 times the amount by weight of methanol is used.

4. Process according to Claim 1, characterized in that ammonia is added in an amount of 0.1 - 8 % by weight, preferably 0.2 - 6 % by weight, relative to the amount of methanol.

5. Process according to Claim 1, characterized in that the waste stream remaining after separating off the ammonium sulphate is fed to a combustion chamber as a low-sulphur waste-fuel.

6. Process according to Claim 1, characterized in that the methanol is distilled off from the waste stream remaining after separating off the ammonium sulphate, together with the ammonia.

7. Process according to Claim 6, characterized in that the methanol, containing ammonia, which is distilled off is used again for the removal of ammonium sulphate from waste streams.

8. Process according to Claim 6, characterized in that the bottom stream from the methanol distillation is kept flowable by addition of water, preferably by addition of water containing ammonia.

9. Process according to Claim 6, characterized in that the waste stream remaining after the methanol distillation is fed to a combustion chamber as a low-sulphur and low-nitrogen waste-fuel.

10. Process according to Claim 1, characterized in that it is carried out continuously.


**Revendications**

1. Procédé pour éliminer le sulfate d'ammonium contenu dans des courants résiduaires à forte teneur en goudrons de la fabrication du (méth)acrylonitrile, obtenus par séparation des liqueurs mères de la cristallisation principale du $(NH_4)_2SO_4$ et qui ont une teneur en goudrons de 25 à 80% en poids, caractérisé en ce que l'on ajoute à ces courants résiduaires, à une température de 10 à 60°C, du méthanol en quantité de 1 à 30 fois le poids de l'eau contenue dans le courant résiduaire, et de 0,05 à 10% en poids d'ammoniac par rapport à la quantité de méthanol, et on sépare le sulfate d'ammonium qui précipite.

2. Procédé selon la revendication 1, caractérisé en ce que l'on opère entre 25 et 45°C.

3. Procédé selon la revendication 1, caractérisé en ce que l'on utilise le méthanol en quantité de 3 à 20 fois le poids de l'eau.

4. Procédé selon la revendication 1, caractérisé en ce que l'on ajoute l'ammoniac en quantité de 0,1 à 8% en poids, de préférence de 0,2 à 6% en poids par rapport au méthanol.

5. Procédé selon la revendication 1, caractérisé en ce que, après séparation du sulfate d'ammonium, le courant résiduaire restant est envoyé en tant que combustible résiduaire à basse teneur en soufre dans une chambre de combustion.

6. Procédé selon la revendication 1, caractérisé en ce que l'on distille le méthanol du courant résiduaire restant avec l'ammoniac après séparation du sulfate d'ammonium.

7. Procédé selon la revendication 6, caractérisé en ce que l'on utilise à nouveau le méthanol distillé contenant de l'ammoniac pour l'élimination du sulfate d'ammonium de courant résiduaire.

8. Procédé selon la revendication 6, caractérisé en ce que l'on maintient le courant de culot de la distillation du méthanol suffisamment fluide par addition d'eau, de préférence par addition d'eau contenant de l'ammoniac.

9. Procédé selon la revendication 6, caractérisé en ce que le courant résiduaire restant après distillation du méthanol est envoyé, en tant que combustible résiduaire à basse teneur en soufre et basse teneur en azote, à une chambre de combustion.

10. Procédé selon la revendication 1, caractérisé en ce qu'il est mis en oeuvre en continu.